# EUROPÄISCHE PATENTANMELDUNG

(11) **EP 0 688 541 A2**
(43) Veröffentlichungstag der Anmeldung: **27.12.1995**
(21) Anmeldenummer: 95109395.4
(22) Anmeldetag: 17.06.1995
(51) Int. Cl.: A61C 13/00, B65D 75/30, B32B 15/08, A61K 6/00

(54) **Bahältnis zur Aufnahme einer Einmal-Dosis eines Zahnprothesen-Haftmittels**

(30) Priorität: 20.06.1994 DE 9409937 U
(71) Anmelder: Hofacker, Erich, D-23684 Pönitz am See (DE)
(72) Erfinder: Hofacker, Erich, D-23684 Pönitz am See (DE)

(57) **Zusammenfassung**

Es wird ein Behältnis zur Aufnahme einer Einmal-Dosis eines Zahnprothesen-Haftmittels beschrieben, die aus einem Viersiegelrandbeutel (10) mit einer mehrschichtigen, feuchtigkeitsdichten rückseitigen Wandung (11) besteht, die entlang ihrer vier Begrenzungskanten mit einer vorderseitigen Wandung (12) aus gleichem Material und im wesentlichen gleicher Größe verschweißt ist.

## Beschreibung

Die Erfindung betrifft ein Behältnis zur Aufnahme einer Einmal-Dosis eines Zahnprothesen-Haftmittels.

Zahnprothesen-Haftmittel kommen als Flüssigkeit, Creme oder Pulver zur Anwendung. Sie werden in entsprechenden Behältnissen, d.h. Flaschen, Tuben oder Streudosen, aufbewahrt und von der die Prothese tragenden Person mitgeführt. Dies führt zu einer Reihe gravierender Nachteile.

Zunächst sind diese Behältnisse relativ groß und führen durch ihr Gewicht und Volumen zu Unannehmlichkeiten, wenn sie in der Tasche getragen werden. Zweitens, und dies ist von außerordentlicher Bedeutung, ist es schwer, die erforderliche Einzeldosierung vorzunehmen. Für die Haftungsdauer der Prothese ist es jedoch von außerordentlicher Bedeutung, das Produkt in ausreichender, jedoch nicht überschüssiger Dosierung aufzubringen. Eine entsprechende Sicherheit ist jedoch nicht gegeben. Schließlich können insbesondere pulverförmige Haftmittel hygroskopisch sein. In größeren Streubehältnissen, die wiederholt feuchter Umgebungsluft ausgesetzt sind, kommt es zur Feuchtigkeitsaufnahme durch das Produkt. Das Pulver backt zusammen, läßt sich schwer oder gar nicht mehr applizieren und ist hinsichtlich seiner Wirksamkeit reduziert.

Angesichts dieser Problematik liegt der Erfindung die Aufgabe zugrunde, ein Behältnis insbesondere für pulverförmige Zahnprothesen-Haftmittel zur Verfügung zu stellen, welches die zuvor aufgezeigten Nachteile vermeidet, d.h., es soll in der Tasche des Prothesenträgers nicht auftragen, es soll eine genaue Einmal-Dosierung gewährleisten sowie eine Feuchtigkeitsversiegelung besitzen, damit ein hygroskopisches Produkt keinerlei Schaden nimmt.

Gelöst wird diese Aufgabe gemäß der Erfindung durch einen Viersiegelrandbeutel mit einer mehrschichtigen feuchtigkeitsdichten, rückseitigen Wandung, die entlang ihrer vier Begrenzungskanten mit einer vorderseitigen Wandung auf gleichem Material mit im wesentlichen gleicher Größe verschweißt ist. Durch diese strukturelle Ausgestaltung lassen sich mit einem Mal alle voranstehend aufgezeigten Probleme lösen. Ein derartiger flacher Viersiegelrandbeutel trägt in der Tasche nicht auf und besitzt ein geringes Gewicht von etwa 2 bis 3 g einschließlich der Einmal-Dosierung des Haftmittels von etwa 2 g. Damit ist auch die exakte Dosierung für den Einmal-Gebrauch gewährleistet. Insbesondere wird durch die Versiegelung ein Feuchtigkeitszutritt aus der Umgebungsluft sicher verhindert, und es tritt keinerlei Veränderung der physikalischen und chemischen Eigenschaften des Pulvers ein.

Gemäß einer bevorzugten Ausführungsform ist das Innenvolumen des Viersiegelrandbeutels einer Menge von 0,5 bis 3,0 g des Haftmittels angemessen. Das bedeutet, daß je nach Wirksamkeit des Haftmittels eine Dosis für den einmaligen Gebrauch von dem Beutel aufgenommen ist. Besonders bevorzugt wird die Menge von etwa 2 g, die bei einem hochwirksamen Haftmittel für eine Einmal-Dosierung ausreicht.

Nach einer zweckmäßigen Ausgestaltung der Erfindung besteht die rückseitige Wandung sowie die vorderseitige Wandung aus einer PETP/ALU/PE-Folie. Dieses Material besitzt den Vorteil, daß es einerseits absolut feuchtigkeitsdicht ist, während andererseits die PE-Beschichtung ein Wärmeschweißverfahren entlang der Peripherie des Beutels gestattet, womit eine feuchtigkeitsdichte Versiegelung gewährleistet ist.

Das Haftmittel besteht vorzugsweise aus hochverdichtetem Kollidon und kann ggf. einen Zusatz aus Vitamin C enthalten. Der Zusatz aus Vitamin C ist besonders wertvoll für ältere Leute, die somit zugleich unter anderem zur Stärkung ihrer Abwehrkräfte mit einer Tagesration versorgt werden. Als besonders bevorzugte Ausgestaltung ist der Viersiegelrandbeutel an seiner äußeren Peripherie mit einer Einreißkerbe versehen. Damit läßt sich der Viersiegelrandbeutel für die Anwendung des Haftmittels ohne zusätzliche Gerätschaften, wie etwa eine Schere, öffnen.

Weitere Vorteile, Einzelheiten und erfindungswesentliche Merkmale ergeben sich aus der nachfolgenden Beschreibung des erfindungsgemäßen Behältnisses, Unter Bezugnahme auf die beigefügten Zeichnungen. Dabei zeigt im einzelnen:
- Fig. 1: die Draufsicht auf eine Viersiegelrandbeutel gemäß der Erfindung und
- Fig. 2: einen Schnitt entlang der Schnittlinie II-II der Fig. 1.

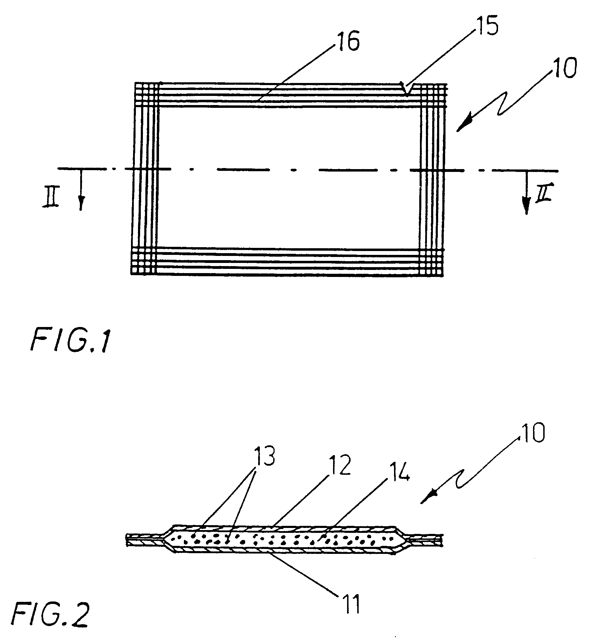

Der in den Fig. 1 und 2 wiedergegebene, in seiner Gesamtheit mit der Bezugsziffer 10 versehene Viersiegelrandbeutel besitzt, wie sich insbesondere aus Fig. 2 ergibt, eine rückseitige Wandung 11 sowie eine vorderseitige Wandung 12. Das Wandungsmaterial ist feuchtigkeitsdicht und wärmeverschweißbar. Eine Verbundfolie aus PETP/ALU/PE erfüllt diese Bedingungen.

Wie sich aus Fig. 2 ergibt, erfolgt die Versiegelung mittels einer fünfrippigen Schweißnaht von insgesamt etwa 5 bis 7 mm Stärke.

Wie in Fig. 2 wiedergegeben ist, befindet sich das Zahnprothesen-Haftmittel 13 innerhalb des Innenraumes 14 zwischen der rückseitigen Wandung 11 und der vorderseitigen Wandung 12 des Viersiegelrandbeutels 10. Das aus hochverdichtetem Kollidon bestehende Haftmittel kann ggf. auch einen Zusatz aus Vitamin C enthalten. Bevorzugte Menge an Vitamin C sind ca. 70 bis 90 mg pro 2 g der Einmal-Dosis des Haftmittels.

An der Peripherie der umlaufenden Schweißversiegelung ist auf einer Seite eine Einreißkerbe 15 angeordnet. Die verbleibenden Schweißrippen 16 der Versiegelung, die von der Einreißkerbe 15 nicht durchgriffen sind, gewährleisten eine hinreichende Abdichtung.

Zur Applikation des Haftmittels trennt der Benutzer den Viersiegelrandbeutel 10 an der Einreißkerbe 15 auf und streut den gesamten Inhalt des Viersiegelrandbeutels 10 an Haftmittel 13 auf die Prothese auf. Damit ist eine Dosierung gewährleistet, und der Beutel 10 kann dem Abfall zugeführt werden.

Es soll an dieser Stelle noch einmal ausdrücklich angegeben werden, daß es sich bei der vorangehenden Beschreibung lediglich um eine solche beispielhaften Charakters handelt und daß verschiedene Abänderungen und Modifikationen möglich sind, ohne dabei den Rahmen der Erfindung zu verlassen.

## Patentansprüche

1. Behältnis zur Aufnahme einer Einmal-Dosis eines Zahnprothesen-Haftmittels,
**gekennzeichnet durch**
einen Viersiegelrandbeutel (10) mit einer mehrschichtigen, feuchtigkeitsdichten rückseitigen Wandung (11), die entlang ihrer vier Begrenzungskanten (16) mit einer vorderseitigen Wandung (12) aus gleichem Material und im wesentlichen gleicher Größe verschweißt ist.

2. Behältnis nach Anspruch 1, dadurch gekennzeichnet, daß das Volumen des Innenraums (14) des Viersiegelrandbeutels (10) einer Menge von 0,5 bis 3,0 g des Haftmittels (13) entspricht.

3. Behältnis nach Anspruch 2, dadurch gekennzeichnet, daß das Volumen des Innenraums (14) des Viersiegelrandbeutels (10) einer Menge von etwa 2 g des Haftmittels (13) entspricht.

4. Behältnis nach einem der vorangehenden Ansprüche, dadurch gekennzeichnet, daß die rückseitige Wandung (11) und die vorderseitige Wandung (12) aus einer PETP/ALU/PE-Folie bestehen.

5. Behältnis nach Anspruch 4, dadurch gekennzeichnet, daß die PE-Beschichtung der Wandungen (11, 12) auf deren jeweiliger Innenseite angeordnet ist.

6. Behältnis nach einem der vorangehenden Ansprüche, dadurch gekennzeichnet, daß das Haftmittel (13) aus hochverdichtetem Kollidon besteht.

7. Behältnis nach einem der vorangehenden Ansprüche, dadurch gekennzeichnet, daß das Haftmittel (13) aus hochverdichtetem Kollidon mit einem Zusatz aus Vitamin C besteht.

8. Behältnis nach einem der vorangehenden Ansprüche, dadurch gekennzeichnet, daß der Viersiegelrandbeutel (10) an seiner äußeren Peripherie (16) mit einer Einreißkerbe (15) versehen ist.
